# EUROPEAN PATENT SPECIFICATION

(11) **EP 4 563 178 B1**
(45) Date of publication and mention of the grant of the patent: **11.11.2026**
(21) Application number: 24216331.9
(22) Date of filing: 29.11.2024
(51) Int. Cl.: A61M 16/20, A61M 16/00, A61M 16/12

(54) **SYSTEM WITH ELECTRICALLY CONTROLLED SOURCE OF GAS STREAM OR PNEUMATIC POWER, WITH CALIBRATED FLOW METER**
SYSTEM MIT ELEKTRISCH GESTEUERTER QUELLE EINES GASSTROMS ODER VON PNEUMATISCHER ENERGIE MIT KALIBRIERTEM DURCHFLUSSMESSER
SYSTÈME À SOURCE DE COURANT GAZEUX OU DE PUISSANCE PNEUMATIQUE COMMANDÉE ÉLECTRIQUEMENT, AVEC DÉBITMÈTRE ÉTALONNÉ

(30) Priority: 01.12.2023 PL 44694823
(43) Date of publication of application: 04.06.2025
(73) Proprietor: Instytut Biocybernetyki i lnzynierii Biomedycznej im. M. Nalecza Polskiej Akademii Nauk, 02-109 Warszawa (PL)
(72) Inventor: KOZARSKI, Maciej, 05-806 Granica (PL); DAROWSKI, Marek, 02-070 Warszawa (PL)
(74) Representative: Patpol Kancelaria Patentowa Sp. z o.o.

(56) References cited:
- EP-A1- 3 936 177

## Description

The invention relates to a system with an electrically controlled source of gas flow or pneumatic power, with a calibrated flow meter and the method of electrically controlling a source of gas flow or pneumatic power. The invention is applicable to devices used in artificial lung ventilation as well as in generators of arbitrary pneumatic waveforms.

Systems with electrically controlled source of gas flow or pneumatic power are the basic functional block in respirators used in situations where there is no access to a central hospital pneumatic supply network. In this context, these systems are called respirator breathing pumps. A respirator equipped with its own breathing pump system is a more complex design solution, but at the same time much more versatile and can be used outside the hospital, e.g. during patient transport or in wartime conditions in a field hospital.

In the art, various designs of the gas flow or pneumatic power sources are known. In particular, in respirators, the gas flow sources may include piston pumps, bellows pumps, vane pumps (turbines), as well as diaphragm pumps. For example, a high-speed diaphragm pump is known from the Polish patent publication P.134717. On the other hand, a low-speed diaphragm pump with an electrical control system including a calibrated flow meter is known from the Polish patent publication PL 195911.

However, it appears that the pumps known from the art and their associated control systems do not ensure continuity and long-term stability of measurement of the breathing gas flow during both the patient's inhalation and exhalation phases. For example, in a gas flow source control system, known from the patent publication PL195911, auto-calibration of the flow meter can be performed no more frequently than every few dozen breaths. There is also known from the patent publication EP3936177 B1 a ventilator for ventilating a patient, wherein the ventilator has a positive pressure unit, in particular a respiratory gas compressor, for providing a respiratory gas pressure flow in or for an inspiratory limb and wherein the positive pressure unit is designed to provide a constant respiratory gas pressure flow, wherein the constant respiratory gas pressure flow can be supplied to the patient at a predetermined time and/or trigger condition.

Similarly, the problem of minimising the resistance of the expiratory path from the selected pressure measurement point all the way to its outlet into the atmosphere remains unresolved. The aim of the invention is to provide a system with an electrically controlled source of gas flow or pneumatic power, which does not have the aforementioned disadvantages indicated in the description of the state of the art.

The invention is defined in the appended claims.

In particular, the aim of the invention is to provide a system with an electrically controlled source of gas flow or pneumatic power with a calibrated flow meter, which would ensure stability of measurement of the breathing gas flow in both breathing phases, but especially in the long term. Secondly, the aim of the invention is to provide a system with an electrically controlled source of gas flow or pneumatic power, which would ensure that the resistance of the expiratory path from the selected pressure measurement point all the way to its outlet to the atmosphere is minimised.

The essence of the disclosure is that a system with an electrically controlled source of gas flow or pneumatic power, comprising a block of calibration source of gas flow or pneumatic power connected pneumatically to the inspiratory section, a flow meter located in the inspiratory section, a microcontroller control block connected to the flow meter, comprising a flow meter calibration module, is characterised in that the flow meter is connected, on one side, between a first check valve of the inspiratory section and an inspiratory tube and its downstream second check valve of the inspiratory section and, on the other side, is connected between a third check valve of an expiratory section, connected to its downstream expiratory tube, and a shut-off valve, so that the flow meter is incorporated diagonally in a bridge arrangement between the four said pneumatic valves, and a calibration flow qi and an expiratory flow qe flow through the flow meter alternately in the inhalation and exhalation phases in the same direction.

Preferably, there is a diverter valve upstream of the first check valve of the inspiratory section, a second output of which is connected to a second input of an ejector, and a first input of the ejector is connected to the output of the shut-off valve, an output of the ejector being connected to the atmosphere via an acoustic attenuator, and the microcontroller control block comprises a breathing valve controller configured to control the diverter valve and the shut-off valve.

Preferably, the calibration source of the gas flow is a diaphragm pump.

Preferably, the microcontroller control block includes an adder which is a circuit for correcting the rotational speed of the gas flow source motor.

Preferably, the mixing valve block comprises at least two diverter valves connected in cascade, each of which has a respective electrical control terminal connected to the mixer valve controller for receiving respective signals uz1 and uz2 controlling the sequential switching of the diverter valves as a function of the angle α of rotation of the pump drive shaft.

Preferably, the input terminals of the block are connected to tanks of gases to be mixed, which gases are expanded to the level of atmospheric pressure.

Preferably, the inspiratory tube and the expiratory tube are connected to an endotracheal tube, which has a breathing pressure measurement output p, connected to the pneumatic terminal of the pressure sensor.

Preferably, an output of the pressure sensor is connected to a measurement input of a pressure control system in the microcontroller control block, the central control programme being configured to supply, during the exhalation phase, to a second input of the pressure control system, an end-expiratory pressure PEEP setpoint signal, and an output of the control system being connected to an input of a signal switch, the output of which is connected to an input of the signal adder, wherein the central control programme is configured to supply, to an input of the signal adder, the signal uec of the correction value taking into account, during the inhalation phase, the compensation of the incoming shunt flow to the intrinsic compliance of the pump and the inspiratory tube during the inhalation phase, and to supply, to an input of a switch, during the inhalation phase, the signal q(t) of the mixture flow generated by the ventilation algorithm module. Preferably, the diaphragm pump has eight sections.

Preferably, the diaphragm pump comprises an encoder for measuring the angle α of rotation of the pump shaft.

Preferably, the block of calibration source of gas flow or pneumatic power comprises a tachometer for measuring the rotational speed n of the motor shaft.

Preferably, the expiratory tube is replaceable.

In another aspect, the essence of the disclosure is that a method of electrically controlling a source of gas flow or pneumatic power comprising generating a gas flow by the source of gas flow or pneumatic power, auto-calibrating the flow meter by a microcontroller control block comprising a flow meter calibration module, measuring the gas flow by the flow meter, is characterised in that in the step of auto-calibrating the flow meter in the inhalation phase, the gas flow qi flows in the auto-calibration block of the flow meter through the flow meter connected between a first check valve of the inspiratory section and an inspiratory tube and a second check valve of the inspiratory section, located downstream thereof, and
- in the step of measuring the gas flow qe, in the exhalation phase, the gas flow qe flows in the auto-calibration block of the flow meter through the flow meter connected between a third check valve of an expiratory section connected to an expiratory tube located downstream thereof and a shut-off valve, so that the calibration flow qi and the expiratory flow qe flow through the flow meter alternately in the inhalation and exhalation phases in the same direction.

The advantage of the system with an electrically controlled source of gas flow or pneumatic power according to the disclosure is that it is self-calibrating thanks to the use of a suitable auto-calibration system for the flow meter in the inhalation phase. To be precise, thanks to the use of an appropriate bridge arrangement, auto-calibration of the flow meter can be performed in each breathing cycle, creating the possibility of an ongoing assessment of flow meter performance.

The system with an electrically controlled source of gas flow or pneumatic power according to the disclosure also comprises a control system to compensate for the resistance of the expiratory path from the exhalation pressure measurement point all the way to its outlet to the atmosphere, using for this purpose a calibration source, which is preferably a multi-segment diaphragm pump.

Furthermore, thanks to the use of the system with an electrically controlled source of gas flow or pneumatic power according to the disclosure, a significant improvement in the functionality of the respirator itself can be achieved, due to the fact that it becomes a low-power electric device that does not require an external pneumatic supply and can also be used in field hospitals, ambulances and treatment rooms.

In addition, thanks to the use of an eight-segment diaphragm pump as a calibration source, exceptional parameter stability of the entire system is achieved.

The system with an electrically controlled source of gas flow or pneumatic power with a calibrated flow meter according to the disclosure is shown in detail in embodiments with reference to the attached drawing figures, in which:
Fig. 1 shows a general diagram of the system with an electrically controlled source of gas flow or pneumatic power according to the invention;
Fig. 2 shows a detailed diagram of the system with an electrically controlled source of gas flow or pneumatic power according to the invention;
Fig. 3 shows a fragment of a cross-section of a diaphragm pump used as a source of gas flow in the system according to the invention;

When carrying out artificial ventilation, knowing the parameters of the gaseous expiratory mixture is as important as knowing the parameters of the inspiration mixture administered. To this end, among other things, the volume of both the administered and exhaled gas mixture is measured using a flow meter. By knowing the volume of the expiratory mixture, it is not only possible to carry out an ongoing monitoring of the correct functioning of the flow meter, but also of the tightness of the system of tubes transporting the breathing gases

An important parameter of the devices used for artificial ventilation is also the resistance of the ventilator's expiratory path, which is usually a significant component of the total expiratory resistance. The existence of expiratory resistance means that the patient is not ventilated optimally, i.e. with the lowest effort, i.e. with the lowest breathing work. To this end, such designs of expiratory paths in the respirator system are sought that minimise the energy loss associated with gas flow.

As mentioned, the system 100 with electrically controlled source of gas flow or pneumatic power according to the disclosure is intended for use in apparatus for conducting artificial ventilation and has been designed to take into account, to the maximum extent possible, the mentioned requirements for stable operation and minimum resistance in the expiratory paths.

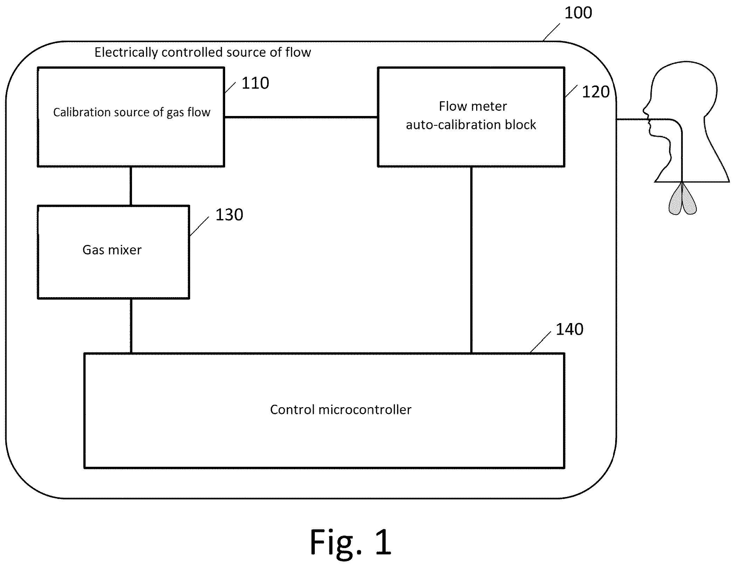

As shown in Fig.1, the system 100 for electrically controlled source of gas flow or pneumatic power according to the disclosure comprises four functional blocks:
- a block 110 of calibration source of gas flow or pneumatic power comprising an electrically controlled source 111 qz of a breathing gas mixture, supplied at a pressure pz;
- a block 120 of gas mixer consisting of cascade-connected, electrically controlled diverter valves 121,122, enabling three gases G1, G2, G3 previously expanded to near atmospheric pressure to be mixed.
- a block 130 for auto-calibration of the flow meter 131, through which the following flow alternately: in the inhalation phase - a precisely known gas flow qi=qz with a known composition, and then in the exhalation phase - a flow qe of exhaled gas with a variable and generally precisely unknown composition, wherein in the inhalation phase
the flow meter 131 is calibrated and in the exhalation phase the exhaled gas flow qe is measured using the already calibrated flow meter 131.
- a microcontroller control block 140, comprising computer applications, i.e. programmes for calculating and controlling the operation of the breathing pump system.

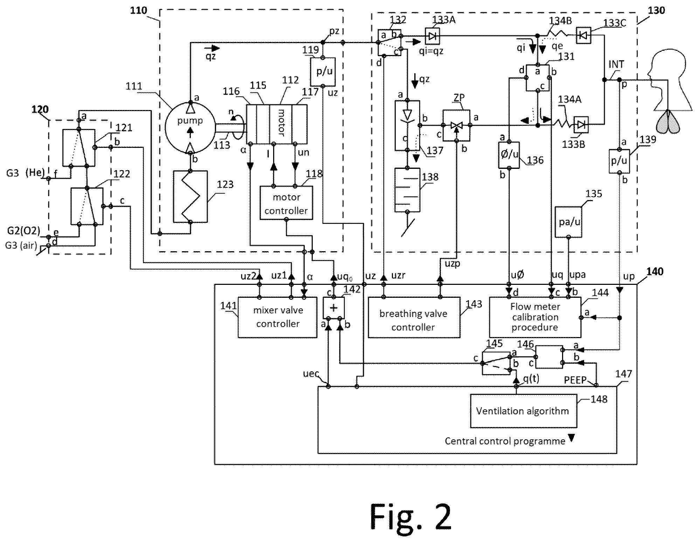

As shown in Fig. 2, the first sub-system of the system 100 for electrically controlled source of gas flow or pneumatic power according to the disclosure is the block 110 of calibration source of gas flow qz, wherein in the context of the respirator, the gas flow is the flow qz of the breathing mixture.

The block 110 of calibration source is characterised in that it comprises a calibration source 111 of flow, in which the flow qz is proportional to the voltage controlling the operation of the source 111. This is an inherent condition of the calibration source 111.

In one embodiment, the calibration source 111 of gas flow is a diaphragm pump. In other embodiments, this could be any other type of pump, assuming that the flow qz coming out of it is proportional to the voltage controlling said pump 110.

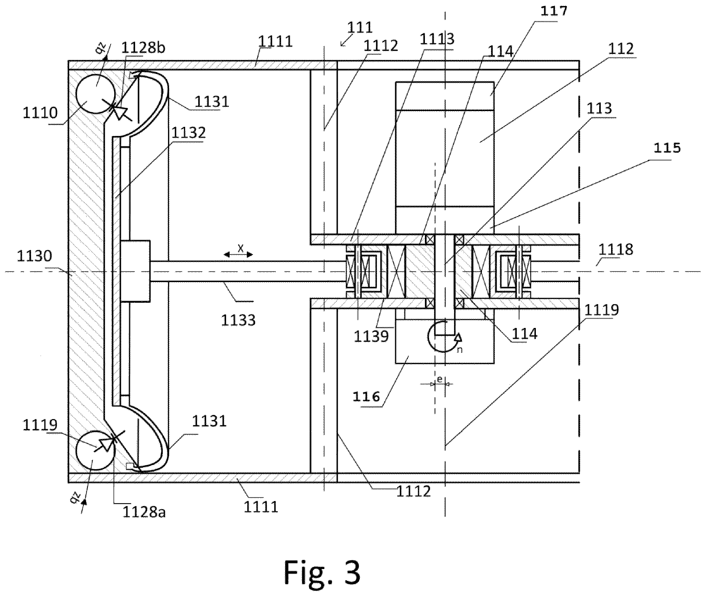

In one embodiment, the calibration source block 110 comprises the calibration source 111 of gas flow or pneumatic power, which is an eight-segment suction and discharge diaphragm pump 111. As shown in Fig. 2, the diaphragm pump 111 is driven by a DC motor 112. The shaft 113 of the pump 111 is connected via a gear 115 to the shaft (not shown in Fig. 3) of the motor 112. The angle α of rotation of the shaft 113 of the pump 111 is measured digitally by an encoder 116.. The rotational speed n of the shaft of the motor 112 is measured analogously by a tachometric generator 117. The rotational speed n of the shaft of the motor 112 is controlled by a controller 118 of the motor 112. A voltage uqₒ is supplied to a control input of the controller 118. The control voltage uqₒ is generated according to appropriate algorithms by appropriate circuits located in the control block 140 of the microcontroller (see ventilation algorithm) depending on the required operating mode of the block 110 of calibration source of gas flow. The controller 118 controls the rotation of the motor unit 112 by supplying a current I so that, as a result, the rotational speed n of the shaft of the motor 112 depends only on the control voltage (i.e. n/un=const.) and is independent of the pressure pz of the discharged gas. Separated from the master control unit 140, the controller 118 of the motor is a typical system that converts an electrical control signal supplied from the control unit 140 of the microcontroller into a current supplied to the windings 112 of the motor.

Details of the design of the diaphragm pump 111 used in one embodiment as the calibration source 111 are shown in Fig. 3, which shows a cross-section through one selected section of the pump 111 out of eight sections in a star arrangement.

The rigid frame of the pump 111 consists of two parts of different widths. In the wider part, it is formed by outer (bottom and top) plates 1111 and, in the narrower part, by inner plates 1113, the outer plates overlapping the inner plates in diameter, so that the outer plates 1111 are fixed rigidly to the inner plates 1113 with a suitable distance by means of posts 1112. Pumping segments 1130 are rigidly mounted between the outer plates 1111. Diaphragms 1131 of preferably variable thickness profile enclose, together with stiffeners 1132, suction and discharge chambers of the pumping segments 1130. Each pumping segment 1130 has suction 1119 and discharge 1110 manifold channels made inside the bodies 1130 of the pumping segments. Inside the bodies 1130 of the pumping segments, there are also check valves 1128a, 1128b indicated with diode symbols.

Between the inner plates 1113 of the pump frame 111, for example, which are only 16 mm apart, there is the entire drive structure of the pumping diaphragms 1131. The drive structure comprises, among others: a floating disc 1139 with bearing-mounted ends of push rods 1133 of the diaphragms 1131 connected to the stiffeners 1132, an eccentric 114, a gear 115 with a bellows clutch mounted on the shaft of the motor unit 112 and a double connecting-rod counter-twist mechanism (not shown) of the floating disc.

The entire drive of the diaphragms 1131 is symmetrical with respect to the axis 1117 of the motor unit 112. The eccentricity e determines the stroke x of the pumping membranes 1113.

The rotational movement of the shaft of the motor 112 induces, through the mechanical gear 115 (preferably a worm gear), the rotational movement of the drive shaft 113 of the pump rigidly connected to the eccentric 114 and, consequently, the reciprocating movement of the stiffeners 1132 of the diaphragms causing the cyclic suction and discharge of gas through the collector ends 111a and 111b, respectively.

By minimising the volume between the inner plates 1113 of the pump 111, additional space was gained in the space of its skeleton, which is located between the outer plates 1111 and the inner plates 1113, to accommodate the motor 112 and, in addition, drive control electronics and a breathing mixture homogeniser (not shown in Fig. 3).

There are many advantages arising from the use of this particular type of calibration source of gas flow. Specifically, the number of eight pumping segments 1130 guarantees a low (lower than 4%) level of flow qz ripple. In addition, the pneumatic, output, intrinsic compliance of the pump 111 (equivalent to the electrical capacity) is practically constant as a function of the pump shaft rotation and can thus be easily taken into account when evaluating the value of the flow qz delivered by the 111 pump. The intrinsic capacity of the 111 pump acts as a pneumatic capacitor, dynamically shunting the output flow qz of the 111 pump. In the case of diaphragm pump 111, the volume of air pumped by the pump 111 is, to a good approximation, proportional to the angle of rotation of the shaft 113 of the pump 111.

It is also very important that the diaphragm pump 111 according to the disclosure can operate continuously in one direction of rotation of the shaft 113 of the pump 111 and can therefore produce positive pressure in the inhalation phase and in the exhalation phase it can be used to supply an ejector which produces negative pressure, thus to build a bipolar gas source.

As shown in Fig. 2, the pump 111 pumps the suctioned breathing mixture into the 130 for auto-calibration of the flow meter 131, producing a flow qz at pressure pz, wherein for pressure pz=0 the flow qz is exactly proportional to the control voltage uq (i.e. qz/uz=const);

On the suction side of the pump 111, there is a subassembly of a mechanical homogeniser 115 of the breathing mixture, which reduces fluctuations in its composition resulting from the volumetric mixing of the sucked portions of the component gases of the mixture. On the discharge side of the pump 111, the pressure pz generated by the pump 111 is measured by a pressure sensor 119, i.e. transducer 119, the electrical output of which provides a voltage uz proportional to the pressure pz of the gas mixture discharged by the pump (uz/pz=const.).

The breathing mixture suctioned by the pump 111 comes from the mixer block 120, which in one embodiment comprises a set of two electro-pneumatic diverter valves 121 and 122, connected in cascade. In other embodiments, the number of diverter valves may be higher. Any three component gases G1, G2, G3 (e.g. air, oxygen, helium) expanded to atmospheric pressure can be supplied to the three free inputs of the valve assembly 121,122. The principle of operation of the mixer block 120 consists in switching on successive inputs d,e,f of the valves 121, 122 in a sequence related to the angle α of rotation of the shaft 113 of the pump 111, resulting in the suction of portions of successive gases G1, G2, G3 in proportion to the length of the respective angular segment of rotation of the shaft 113 of the pump 111. This is a method of very accurate, volumetric mixing of components, completely independent of the momentary flow qz of the breathing gas. The inputs d,e,f of the valves 121, 122 are controlled by the microcontroller control block 140, which comprises, for this purpose, an appropriate controller 141 of the mixer valves. This controller obtains the value of the angle α of rotation of the shaft 113 of the pump 111 from the block 110 of calibration source of gas flow or pneumatic power.

As mentioned, the pump 111 pumps the gas flow qz to the inlet diverter valve 131 of the block 130 for auto-calibration of the flow meter 131.

The structure of the block 130 for auto-calibration of the flow meter 131 enables two key tasks to be carried out:
- auto-calibration of the breathing flow meter 131,
- compensation of the expiratory path resistance from the selected breathing pressure measurement point p all the way to the outlet to the atmosphere.

As shown in Fig. 2, in the block 130, the arrows indicating the directions of the breathing mixture flows are drawn with a solid line for the inhalation phase and a dashed line for the exhalation phase.

During the inhalation phase, the flow qi=qz is directed through the diverter valve 132 and then a first check valve 133A to the flow meter 131, and further through an inspiratory tube 134A, through a second check valve 133B and an endotracheal tube INT into the patient's lungs. The function of the first check valve 133A, the second check valve 133B and a third check valve 133C, is to allow the gas to flow in one direction only, indicated by the diode symbol, so that the constant direction of gas flow through the flow meter 131 is always under control.

In the exhalation phase, the flow qe exhaled by the patient is directed through the third check valve 133C, an expiratory tube 134B and a shut-off valve ZP into the suction channel of an ejector 137. The third check valve 133C connects the patient's airways to the flow meter 131 and, via this flow meter 131, the shut-off valve ZP and the ejector 137, connects them to the atmosphere. The third check valve 133C cuts off the flow to the atmosphere during the inhalation phase and allows flow to the atmosphere during the exhalation phase. The function of the shut-off valve ZP is to cut off the gas flow when gas is flowing during the inhalation phase, i.e. without the use of the shut-off valve ZP the breathing gas flow would be directed almost entirely to the atmosphere and not to the patient.

Compensation of the inspiratory path resistance from the selected breathing pressure measurement point p all the way to the outlet to the atmosphere is made possible by the following system. The selected breathing pressure measurement point p should be understood as any pressure measurement point along the expiratory path. In the exhalation phase, the ejector 137 is fed with the flow qz supplied by the calibration source 111 of the gas flow, for example the diaphragm pump 111, via the second output of the diverter valve 132. The outlet of the ejector 137 is connected to the atmosphere via an acoustic damper 138. The function of the ejector 137 is to create a negative pressure, which is achieved by automatically selecting the rotational speed set by a speed control system 146.

During the exhalation phase, the diaphragm pump 111 operates with a waveform additionally imposed by the speed control system 146 located in the microcontroller control block 140. As input, the pump speed control system 146 receives the measured value of pressure p of the expiratory mixture, e.g. in the endotracheal tube INT, in the form of a voltage up from the pressure sensor 139, and the set value of the expected expiratory pressure PEEP given by a ventilation algorithm 148 which is a module of a central control programme 147. The rotational speed of the pump 111 is set by the control system 146 at an instantaneous level n, which causes the negative pressure generated by the ejector 137 to bring the expiratory pressure p to the set PEEP level. For example, assuming PEEP=0 means introducing a so-called "artificial atmosphere" at the pressure measurement point. In any case, from the patient's point of view, this means reducing the resistance of the expiratory path to zero along the entire section starting from the pressure measurement point p.

In the block 130 for auto-calibration of the flow meter, the flow meter 131 is connected, on one side, between the first check valve 133A of the inspiratory section and the inspiratory tube 134A and its downstream second check valve 133B of the inspiratory section and, on the other side, is connected between the third check valve 133C of the expiratory section connected to its downstream expiratory tube 134B and the shut-off valve ZP, so that the flow meter 131 is located on the diagonal of a pneumatic bridge, and the calibration flow qi and the expiratory flow qe flow through the flow meter 131 alternately in the inhalation and exhalation phases in the same direction.

The flow meter 131 is incorporated diagonally in a bridge arrangement between the four said pneumatic valves. As a result, a gas flow of known composition and value flows through the flow meter 131 during the inhalation phase. In this phase, the flow meter 131 is calibrated. In the exhalation phase, an exhaled air flow flows through the same flow meter 131 and in the same direction. In this phase, measurement is performed with the flow meter 131.

In other words, the bridge arrangement in which the flow meter 131 is incorporated ensures the same flow direction through the flow meter 131 of the discharged flow qi and the expiratory flow qe. As a result, the flow meter 131 can be checked and calibrated in each inhalation phase (qi=qz and gas composition are known) while the flow qe of the exhaled gas can be measured accurately in the exhalation phase.

The procedure for calibrating the flow meter 131 is performed by a calibration module 144 of the flow meter contained in the microcontroller control block 140. The following are fed to its input from the block 130 for auto-calibration of the flow meter 131: the temperature value Θ measured by a temperature sensor 136, the flow value qi, the barometric pressure value measured by a pressure sensor 135, the breathing pressure value p measured by a pressure sensor 139. Measurements of temperature Θ and barometric pressure pa are necessary in order to, in a generally known way, normalise the measurement results of the flows qi and qe.

The microcontroller control block 140 is the last block of the system with an electrically controlled source of gas flow according to the disclosure.

The microcontroller control block 140 is built on the basis of a microcontroller performing measurement and control functions. Fig. 2 shows its basic tasks in a block manner. The obvious data acquisition and transfer functions taking place via ADCs and DACs and digital ports are omitted. The control and measurement functions are implemented using a computer application residing in the microcontroller memory. It includes modules such as:
- a controller 141 of the valves of the mixer block 120, which is an autonomous subroutine controlling the operation of the valves 121 and 122 as a function only of the angle α of rotation of the shaft of the pump 111, and produces voltage signals uz1 and uz2 switching them on sequentially,
- a controller 143 of the breathing valves 132 and ZP of the block 130 for auto-calibration of the flow meter, which switches on the pneumatic paths of the valves 132 and ZP in the rhythm of the breathing phases, sending the voltage signals uzr and uzp respectively,
- the module 144 for calibration of the flow meter 131, which performs, in successive breathing cycles, a comparison of the characteristics of the output voltage uq of the flow meter 131 as a function of the calibrating flow qz= qi of the inspiratory gas, at the same time calibrating the flow meter 131 on an ongoing basis and assessing its long-term stability (illustrating the degree of pollution), and normalises the gas flow measurement, according to the generally known thermodynamic relations, on the basis of the signal uΘ of the temperature Θ in the measuring head of the flow meter 131 and the signal ua of the barometric pressure pa and the signal up of the breathing pressure p.

The automatic calibration system 144 eliminates the influence of long-term drifts in both zero and gain of the flow meter 131 of exhaled air.

The microcontroller control block 140 also comprises the speed control system 146 for the pump 111, the inputs of which are supplied with the signals up of the breathing pressure p and the end-expiratory pressure PEEP. The control system 146 comprising a PID-type controller 146, during the inhalation phase, through a software switch 145, sends a signal controlling the pump 111, modified by an adder 142 through an ongoing addition of the calculated correction uec. This correction takes into account the fact that pump 111 has a certain, intrinsic, practically constant, output pneumatic compliance which shunts the dynamically generated flow qz. During the inhalation phase, the flow signal q(t) produced by the algorithm of the selected method of artificial lung ventilation is fed to the software switch 145, which sends the signal controlling the operation of the pump 111. This could be any of the known ventilation methods. In particular, the used source 111 of gas flow, i.e. pump 111, is a universal component in the sense that it can be used in very different ways depending on the type of feedback used in the system controlling the respirator or laboratory generator.

The use of feedback from the rotational speed of the source motor shaft and thus of the pump drive shaft 113 leads to the construction of a controlled flow source 111, i.e. for a specific setpoint flow value of the control system in the outlet channel of the pump 111, a flow rate is obtained that is proportional to this value and independent of the pressure in the pump discharge manifold.

The use of external feedback from the pressure in the discharge manifold or from the pressure p measured by means of an electromanometer 139 (transducer 139) in the tubes 134A and 134B or alternatively measured at a selected point of the endotracheal tube INT leads to a system maintaining a pressure value at the point of measurement that is independent of the gas flow and proportional to the set value of this pressure, i.e. to a so-called controlled pressure source system. In turn, the feedback maintaining a constant value of the product of pressure and flow rate leads to the construction of a controlled pneumatic power source system.

The operation of the system according to the disclosure in the inhalation phase therefore consists in controlling the operation of the described pump 111, which in its output pneumatic channel 111a provides a flow rate proportional to the rotational speed of the electric motor 112, according to an algorithm maintaining a set value of a selected breathing parameter, for example gas flow, pressure or breathing power.

In Fig. 2, in the control block 140, the central control programme 148 is symbolically separated, which synchronises the actions of all functional blocks in the rhythm of breathing cycles.

The only exception to this rule is the valve controller 141 of the mixer block 120, which operates autonomously and is started at the beginning of the first cycle of the system operation.

The control block 140 is further configured for the acquisition of measurement data, control procedures for the operation of other functional blocks, algorithms realising selected ventilation modes, the acquisition of artificial ventilation operating parameters set by the physician.

The developed system with an electrically controlled source of gas flow or pneumatic power, in the first place, can be used in the construction of respirators for long-term lung ventilation, intended for intensive care units, and directly applied to experiments involving animals.

Furthermore, an electrically controlled source of gas flow, pressure or pneumatic power with a calibrated flow meter can be used in laboratories as an element of an arbitrary pneumatic waveform generator, e.g. for testing the measuring heads of flow meters and spirometers.

It should also be emphasised that all elements of the flow paths that come into contact with the air exhaled by the patient, in particular the expiratory tube 134B, can be made in a disposable version, which seems necessary in the light of epidemic experiences.

## Claims

1. A system with an electrically controlled source of gas flow or pneumatic power, comprising
- a block (110) of calibration source (111) of gas flow or pneumatic power connected pneumatically to an inspiratory section,
- a flow meter (131) located in the inspiratory section,
- a microcontroller control block (140) connected to the flow meter (131), comprising a flow meter calibration module (144),
the flow meter (131) being connected, on one side, between a first check valve (133A) of the inspiratory section and an inspiratory tube (134A) and its downstream second check valve (133B) of the inspiratory section and, on the other side, is connected between a third check valve (133C) of an expiratory section, connected to its downstream expiratory tube (134B), and a shut-off valve (ZP), so that the flow meter (131) is incorporated diagonally in a bridge arrangement between the four said pneumatic valves **characterised in that** a calibration flow qi and an expiratory flow qe flow through the flow meter (131) alternately in the inhalation and exhalation phases in the same direction.

2. The system according to claim 1, **characterised in that** there is a diverter valve (132) upstream of the first check valve (133A) of the inspiratory section, a second output (132c) of which is connected to a second input (137a) of an ejector (137), and a first input (137b) of the ejector is connected to an output (ZPc) of the shut-off valve (ZP), an output (137c) of the ejector (137) being connected to the atmosphere via an acoustic attenuator (138), and the microcontroller control block (140) comprises a breathing valve controller (143) configured to control the diverter valve (132) and the shut-off valve (ZP).

3. The system according to claim 2, **characterised in that** the calibration source (111) of gas flow is a diaphragm pump (111).

4. The system according to claim 2 or claim 3, **characterized in that** the microcontroller control unit (140) comprises an adder (142) being a system for correcting the rotational speed of the motor of the gas flow source (111).

5. The system according to claim 1 or 2 or 3 or 4, **characterised in that** a mixing valve block (120) comprises at least two diverter valves (121,122) connected in cascade, each of which has a respective electrical control terminal (121b,122c) connected to a mixer valve controller (141) for receiving respective signals uz1 and uz2 controlling the sequential switching of the diverter valves (121,122) as a function of the angle α of rotation of a drive shaft (113) of the pump (111).

6. The system according to claim 5, **characterised in that** input terminals (120d,120e,120f) of the block (120) are connected to tanks of gases to be mixed, which gases are expanded to the level of atmospheric pressure.

7. The system according to any of claims 1 to 6, **characterised in that** the inspiratory tube (134B) and the expiratory tube (134B) are connected to an endotracheal tube (INT), which has a breathing pressure measurement output p, connected to a pneumatic terminal (139a) of a pressure sensor (139).

8. The system according to claim 7, **characterised in that** an output of the pressure sensor (139) is connected to a measurement input (146a) of a pressure control system (146) in the microcontroller control block (140), the central control programme (147) being configured to supply, during the exhalation phase, to a second input (146b) of the pressure control system (146), an end-expiratory pressure PEEP setpoint signal, and an output (146c) of the control system (146) being connected to an input (145b) of a signal switch (145), the output (145c) of which is connected to an input (142b) of the signal adder (142), wherein the central control programme (147) is configured to supply, to an input (142a) of the signal adder (142), the signal uec of the correction value taking into account, during the inhalation phase, the compensation of the incoming shunt flow to the intrinsic compliance of the pump (111) and the inspiratory tube (134A) during the inhalation phase, and to supply, to an input (142a) of a switch (142), during the inhalation phase, the signal q(t) of the mixture flow generated by the ventilation algorithm module.

9. The system according to claim 3, **characterised in that** the diaphragm pump (111) has eight sections.

10. The system according to claim 3, **characterised in that** the diaphragm pump comprises an encoder (116) for measuring the angle α of rotation of the shaft (113) of the pump (111).

11. The system according to claim 3, **characterised in that** the block of calibration source of gas flow or pneumatic power comprises a tachometer (117) for measuring the rotational speed n of the shaft of the motor (112).

12. The system according to any of claims 1 to 11, **characterised in that** the expiratory tube (134B) is replaceable.

13. A method of electrically controlling a source of gas flow or pneumatic power, comprising
- generating a gas flow by the source (111) of gas flow or pneumatic power,
- auto-calibration of a flow meter (131) by a microcontroller control block (140) comprising a flow meter calibration module (144),
- measuring the gas flow through the flow meter (131),
**characterised in that**
- in the step of auto-calibrating the flow meter, the gas flow qi flows in the flow meter auto-calibration block (130) through the flow meter (131) connected between a first check valve (133A) of the inspiratory section and an inspiratory tube (134A) and a second check valve (133B) of the inspiratory section, located downstream thereof, and
- in the step of measuring the gas flow qe, the gas flow qe flows in the flow meter auto-calibration block (130) through the flow meter (131) connected between a third check valve (133C) of an expiratory section connected to an expiratory tube (134B), located downstream thereof, and a shut-off valve (ZP),
so that the calibration flow qi and the expiratory flow qe flow through the flow meter (131) alternately in the step of auto-calibrating and the step of measuring the gas flow qe in the same direction.

## Patentansprüche

1. System mit einer elektrisch geregelten Quelle für Gasfluss oder pneumatische Leistung, umfassend
- einen Block (110) mit einer Kalibrierungsquelle (111) für Gasfluss oder pneumatische Leistung, der pneumatisch an einen Inspirationsabschnitt angeschlossen ist,
- einen Durchflussmesser (131), der im Inspirationsabschnitt angeordnet ist,
- einen an den Durchflussmesser (131) angeschlossenen Mikrocontroller-Steuerblock (140), umfassend ein Durchflussmesser-Kalibrierungsmodul (144),
**dadurch gekennzeichnet, dass** der Durchflussmesser (131) auf der einen Seite zwischen einem ersten Rückschlagventil (133A) des Inspirationsabschnitts und einem Inspirationsschlauch (134A) sowie dessen stromabwärts gelegenem zweiten Rückschlagventil (133B) des Inspirationsabschnitts angeschlossen ist und auf der anderen Seite zwischen einem dritten Rückschlagventil (133C) eines Exspirationsabschnitts, das an dessen stromabwärts gelegenen Exspirationsschlauch (134B) angeschlossen ist, und einem Absperrventil (ZP) angeschlossen ist, sodass der Durchflussmesser (131) diagonal in einer Brückenanordnung zwischen den vier genannten pneumatischen Ventilen eingebunden ist und ein Kalibrierungsfluss qi sowie ein Exspirationsfluss qe in der Ein- und Ausatmungsphase abwechselnd in derselben Richtung durch den Durchflussmesser (131) fließen.

2. System nach Anspruch 1, **dadurch gekennzeichnet, dass** stromaufwärts des ersten Rückschlagventils (133A) des Inspirationsabschnitts ein Umschaltventil (132) vorliegt, dessen zweiter Ausgang (132c) an einen zweiten Eingang (137a) eines Ejektors (137) angeschlossen ist, und ein erster Eingang (137b) des Ejektors an einen Ausgang (ZPc) des Absperrventils (ZP) angeschlossen ist, wobei ein Ausgang (137c) des Ejektors (137) über einen Schalldämpfer (138) mit der Atmosphäre verbunden ist, und der Mikrocontroller-Steuerblock (140) eine Atemventilsteuerung (143) umfasst, die zur Steuerung des Umschaltventils (132) und des Absperrventils (ZP) ausgelegt ist.

3. System nach Anspruch 2, **dadurch gekennzeichnet, dass** die Kalibrierungsquelle (111) für den Gasfluss eine Membranpumpe (111) ist.

4. System nach Anspruch 2 oder Anspruch 3, **dadurch gekennzeichnet, dass** die Mikrocontroller-Steuereinheit (140) einen Addierer (142) umfasst, der ein System zum Korrigieren der Drehzahl des Motors der Gasflussquelle (111) ist.

5. System nach Anspruch 1 oder 2 oder 3 oder 4, **dadurch gekennzeichnet, dass** ein Mischventilblock (120) mindestens zwei in Kaskade geschaltete Umschaltventile (121, 122) umfasst, von denen jedes einen jeweiligen elektrischen Steueranschluss (121b, 122c) aufweist, die an eine Mischventilsteuerung (141) angeschlossen sind, um jeweilige Signale uz1 und uz2 zu empfangen, die das sequenzielle Schalten der Umschaltventile (121, 122) in Abhängigkeit vom Drehwinkel α einer Antriebswelle (113) der Pumpe (111) steuern.

6. System nach Anspruch 5, **dadurch gekennzeichnet, dass** die Eingangsanschlüsse (120d, 120e, 120f) des Blocks (120) an Behälter für die zu mischenden Gase angeschlossen sind, wobei diese Gase auf Atmosphärendruckniveau entspannt werden.

7. System nach irgendeinem der Ansprüche 1 bis 6, **dadurch gekennzeichnet, dass** der Inspirationsschlauch (134B) und der Exspirationsschlauch (134B) mit einem Endotrachealtubus (INT) verbunden sind, der einen Ausgang p zur Messung des Atemdrucks aufweist, der an einen pneumatischen Anschluss (139a) eines Drucksensors (139) angeschlossen ist.

8. System nach Anspruch 7, **dadurch gekennzeichnet, dass** ein Ausgang des Drucksensors (139) an einen Messeingang (146a) eines Druckregelsystems (146) im Mikrocontroller-Steuerblock (140) angeschlossen ist, wobei das zentrale Regelprogramm (147) ausgelegt ist, um während der Ausatmungsphase an einen zweiten Eingang (146b) des Druckregelsystems (146) ein PEEP-Sollwertsignal für den endexspiratorischen Druck zu liefern, und wobei ein Ausgang (146c) des Regelsystems (146) an einen Eingang (145b) eines Signalumschalters (145) angeschlossen ist, dessen Ausgang (145c) an einen Eingang (142b) des Signaladdierers (142) angeschlossen ist, wobei das zentrale Regelprogramm (147) ausgelegt ist, um an einen Eingang (142a) des Signaladdierers (142) das Signal uec des Korrekturwerts an einen Eingang (142a) des Signaladdierers (142) zu liefern, wobei während der Einatmungsphase die Kompensation des eintreffenden Shunt-Flusses gegenüber der intrinsischen Compliance der Pumpe (111) und des Inspirationsschlauchs (134A) berücksichtigt wird, und während der Einatmungsphase an einen Eingang (142a) eines Schalters (142) das vom Beatmungsalgorithmusmodul erzeugte Signal q(t) des Mischungsstrom zu liefern.

9. System nach Anspruch 3, **dadurch gekennzeichnet, dass** die Membranpumpe (111) acht Segmente aufweist.

10. System nach Anspruch 3, **dadurch gekennzeichnet, dass** die Membranpumpe einen Encoder (116) zum Messen des Drehwinkels α der Welle (113) der Pumpe (111) umfasst.

11. System nach Anspruch 3, **dadurch gekennzeichnet, dass** der Block mit der Kalibrierungsquelle für Gasfluss oder pneumatische Leistung einen Drehzahlmesser (117) zum Messen der Drehzahl n der Welle des Motors (112) umfasst.

12. System nach irgendeinem der Ansprüche 1 bis 11, **dadurch gekennzeichnet, dass** der Exspirationsschlauch (134B) austauschbar ist.

13. Verfahren zur elektrischen Regelung einer Quelle für Gasfluss oder pneumatische Leistung, umfassend
- Erzeugung eines Gasflusses durch die Quelle für Gasfluss oder pneumatische Leistung,
- Selbstkalibrierung eines Durchflussmessers durch einen Mikrocontroller-Steuerblock, umfassend ein Durchflussmesser-Kalibrierungsmodul,
- Messung des Gasflusses durch den Durchflussmesser,
**dadurch gekennzeichnet, dass**
- im Schritt der Selbstkalibrierung des Durchflussmessers der Gasfluss qi in den Durchflussmesser-Selbstkalibrierungsblock (130) durch den Durchflussmesser (131) fließt, der zwischen einem ersten Rückschlagventil (133A) des Inspirationsabschnitts und einem Inspirationsschlauch (134A) sowie einem zweiten, stromabwärts davon angeordneten Rückschlagventil (133B) des Inspirationsabschnitts angeschlossen ist, und
- im Schritt der Messung des Gasflusses qe der Gasfluss qe in den Durchflussmesser-Selbstkalibrierungsblock (130) durch den Durchflussmesser (131) fließt, der zwischen einem dritten Rückschlagventil (133C) eines Exspirationsabschnitts, der an einen stromabwärts davon angeordneten Exspirationsschlauch (134B) angeschlossen ist, und einem Absperrventil (ZP) angeschlossen ist,
sodass der Durchflussmesser (131) diagonal in eine Brückenanordnung zwischen den vier genannten pneumatischen Ventilen eingebunden ist und der Kalibrierungsfluss qi sowie der Exspirationsfluss qe im Schritt der Selbstkalibrierung und im Schritt der Messung des Gasflusses qe abwechselnd in derselben Richtung durch den Durchflussmesser (131) fließen.

## Revendications

1. Système doté d'une source de flux de gaz ou de puissance pneumatique à commande électrique, comprenant
- un bloc (110) de source d'étalonnage (111) du flux de gaz ou de la puissance pneumatique, relié par voie pneumatique à une section inspiratoire,
- un débitmètre (131) situé dans la section inspiratoire,
- un bloc de commande à microcontrôleur (140) relié au débitmètre (131), comprenant un module d'étalonnage de débitmètre (144),
**caractérisé en ce que** le débitmètre (131) est raccordé, d'un côté, entre un premier clapet anti-retour (133A) de la section inspiratoire et un tube inspiratoire (134A) et son deuxième clapet anti-retour aval (133B) de la section inspiratoire et, de l'autre côté, est raccordé entre un troisième clapet anti-retour (133C) d'une section expiratoire, relié à son tube expiratoire aval (134B), et une vanne d'arrêt (ZP), de sorte que le débitmètre (131) est intégré en diagonale dans un montage en pont entre les quatre vannes pneumatiques susmentionnées, et qu'un flux d'étalonnage qi et un flux expiratoire qe traversent le débitmètre (131) alternativement pendant les phases d'inspiration et d'expiration, dans le même sens.

2. Système selon la revendication 1, **caractérisé en ce qu'**une vanne de dérivation (132) est disposée en amont du premier clapet anti-retour (133A) de la section inspiratoire, dont une deuxième sortie (132c) est reliée à une deuxième entrée (137a) d'un éjecteur (137), et une première entrée (137b) de l'éjecteur est reliée à une sortie (ZPc) de la vanne d'arrêt (ZP), une sortie (137c) de l'éjecteur (137) étant reliée à l'atmosphère via un atténuateur acoustique (138), et le bloc de commande à microcontrôleur (140) comprend un contrôleur de vanne respiratoire (143) configuré pour commander la vanne de dérivation (132) et la vanne d'arrêt (ZP).

3. Système selon la revendication 2, **caractérisé en ce que** la source d'étalonnage (111) du flux de gaz est une pompe à membrane (111).

4. Système selon la revendication 2 ou la revendication 3, **caractérisé en ce que** l'unité de commande à microcontrôleur (140) comprend un additionneur (142) constituant un système de correction de la vitesse de rotation du moteur de la source de flux de gaz (111).

5. Système selon la revendication 1, 2, 3 ou 4, **caractérisé en ce qu'**un bloc de vannes de mélange (120) comprend au moins deux vannes de dérivation (121, 122) montées en cascade, dont chacune comporte une borne de commande électrique respective (121b, 122c) reliée à un contrôleur de vannes de mélange (141) pour recevoir des signaux respectifs uz1 et uz2 commandant la commutation séquentielle des vannes de dérivation (121, 122) en fonction de l'angle α de rotation d'un arbre d'entraînement (113) de la pompe (111).

6. Système selon la revendication 5, **caractérisé en ce que** les bornes d'entrée (120d, 120e, 120f) du bloc (120) sont raccordées à des réservoirs contenant les gaz à mélanger, lesquels gaz sont détendus jusqu'à la pression atmosphérique.

7. Système selon l'une quelconque des revendications 1 à 6, **caractérisé en ce que** le tube inspiratoire (134B) et le tube expiratoire (134B) sont raccordés à un tube endotrachéal (INT), qui comporte une sortie de mesure de pression respiratoire p, reliée à une borne pneumatique (139a) d'un capteur de pression (139).

8. Système selon la revendication 7, **caractérisé en ce qu'**une sortie du capteur de pression (139) est reliée à une entrée de mesure (146a) d'un système de contrôle de pression (146) dans le bloc de commande à microcontrôleur (140), le programme de commande central (147) étant configuré pour fournir, pendant la phase d'expiration, à une deuxième entrée (146b) du système de contrôle de pression (146), un signal de consigne de pression de fin d'expiration (PEEP), et une sortie (146c) du système de contrôle (146) étant reliée à une entrée (145b) d'un commutateur de signaux (145), dont la sortie (145c) est reliée à une entrée (142b) de l'additionneur de signaux (142), le programme de commande central (147) étant configuré pour fournir, à une entrée (142a) de l'additionneur de signaux (142), le signal uec de la valeur de correction tenant compte, pendant la phase d'inspiration, de la compensation du flux de dérivation entrant par rapport à la compliance intrinsèque de la pompe (111) et du tube inspiratoire (134A) pendant la phase d'inspiration, et à fournir, à une entrée (142a) d'un commutateur (142), pendant la phase d'inspiration, le signal q(t) du flux de mélange généré par le module d'algorithme de ventilation.

9. Système selon la revendication 3, **caractérisé en ce que** la pompe à membrane (111) comporte huit sections.

10. Système selon la revendication 3, **caractérisé en ce que** la pompe à membrane comprend un codeur (116) destiné à mesurer l'angle α de rotation de l'arbre (113) de la pompe (111).

11. Système selon la revendication 3, **caractérisé en ce que** le bloc de source d'étalonnage du flux de gaz ou de la puissance pneumatique comprend un tachymètre (117) destiné à mesurer la vitesse de rotation n de l'arbre du moteur (112).

12. Système selon l'une quelconque des revendications 1 à 11, **caractérisé en ce que** le tube expiratoire (134B) est remplaçable.

13. Procédé de commande électrique d'une source de flux de gaz ou de puissance pneumatique, comprenant
- génération d'un flux de gaz à l'aide d'une source de flux de gaz ou de puissance pneumatique,
- étalonnage automatique d'un débitmètre par un bloc de commande à microcontrôleur comprenant un module d'étalonnage de débitmètre,
- mesure du flux de gaz à l'aide du débitmètre,
**caractérisé en ce que**
- lors de l'étape d'étalonnage automatique du débitmètre, le flux de gaz qi circule dans le bloc d'étalonnage automatique de débitmètre (130) à travers le débitmètre (131) relié entre un premier clapet anti-retour (133A) de la section inspiratoire et un tube inspiratoire (134A) et un deuxième clapet anti-retour (133B) de la section inspiratoire, situé en aval de celui-ci, et
- lors de l'étape de mesure du flux de gaz qe, le flux de gaz qe traverse le bloc d'étalonnage automatique de débitmètre (130) à travers le débitmètre (131) raccordé entre un troisième clapet anti-retour (133C) d'une section expiratoire reliée à un tube expiratoire (134B), situé en aval de celui-ci, et une vanne d'arrêt (ZP), de sorte que le débitmètre (131) soit intégré en diagonale dans un montage en pont entre les quatre vannes pneumatiques susmentionnées, et que le flux d'étalonnage qi et le flux expiratoire qe traversent le débitmètre (131) alternativement, lors de l'étape d'étalonnage automatique et de l'étape de mesure du débit de gaz qe, dans le même sens.
